**Europäisches Patentamt**

**European Patent Office**

**Office européen des brevets**

(19)

(11) Veröffentlichungsnummer: **0 113 832**
**A1**

(12) # EUROPÄISCHE PATENTANMELDUNG

(21) Anmeldenummer: **83111542.3**

(51) Int. Cl.³: **A 61 N 5/06,** A 47 C 27/00

(22) Anmeldetag: **18.11.83**

(30) Priorität: **15.12.82 DE 3246436**

(43) Veröffentlichungstag der Anmeldung: **25.07.84**
**Patentblatt 84/30**

(84) Benannte Vertragsstaaten: **AT BE CH DE FR GB IT LI LU NL SE**

(71) Anmelder: **Rey, Alfred, Maweestrasse 7, CH-9014 St. Gallen (CH)**

(72) Erfinder: **Rey, Alfred, Maweestrasse 7, CH-9014 St. Gallen (CH)**

(74) Vertreter: **Riebling, Günter Dr.-Ing., Dipl.-Ing., Ing.grad. et al, Rennerle 10 Postfach 3160, D-8990 Lindau (DE)**

(54) **Liegefläche mit Solarium-Bestrahlung.**

(57) Die Liegefläche mit Solarium-Bestrahlung besteht aus einem höhenverstellbaren Solarhimmel (14), der über einer Liegematte (5) angeordnet ist. Die Liegematte besteht hierbei aus einer schwimmenden Fläche, die leicht beweglich die Abdichtung einer mit Flüssigkeit gefüllten Wanne (1) bildet. Der auf der Liegematte aufliegende Benutzer schwimmt trocken auf der Wasseroberfläche und wird von oben von dem Solarhimmel bestrahlt.

EP 0 113 832 A1

## Liegefläche mit Solarium-Bestrahlung

Die Erfindung betrifft eine Liegefläche mit Solarium-Bestrahlung, wobei ein Solarhimmel höhenverstellbar über einer Liegematte mit etwa rechteckigem Grundriss angeordnet ist. Eine derartige Liegefläche ist in vielfältigen Ausführungsformen bekannt geworden. Allen Ausführungsformen ist gemeinsam, daß die Liegefläche als Holzrost fest auf einem ortsfesten Gestell angeordnet ist und dadurch relativ unbequem ist. Überdies ist der durch das Liegen auf der Liegefläche gewonnene Erholungseffekt nur geringfügig.

Die vorliegende Erfindung hat sich die Aufgabe gestellt, eine Liegefläche mit Solarium-Bestrahlung der eingangs genannten Art so weiterzubilden, daß der Erholungseffekt bei der Solarium-Bestrahlung wesentlich verbessert und erweitert ist.

Zur Lösung der gestellten Aufgabe ist die Erfindung dadurch gekennzeichnet, daß die Liegematte als schwimmende Fläche abgedichtet und leicht beweglich die Abdeckung einer mit Flüssigkeit gefüllten Wanne bildet.

Mit der gegebenen technischen Lehre wird also ein ganz neuer Weg beschritten, indem die Liegefläche nun nicht mehr als Holzrost ortsfest auf einem Gestell angeordnet ist, sondern die Schwimmfläche einer mit Flüssigkeit gefüllten Wanne bildet. Diese Liegematte ist rundum abgedichtet am äußeren, umlaufenden Rand der Wanne befestigt, so daß die darauf liegende Person einen Liegeeffekt erhält, wie er etwa mit einer Gummimatte erreicht wird, die auf dem Mittelmeer schwimmt. Die von oben her einwirkende Solarium-Bestrahlung wird an der Oberfläche der mit Flüssigkeit gefüllten Wanne reflektiert, sofern die Liegematte durchsichtig ist und die darin angeordneten Schwimmkörper ebenfalls durchsichtig ausgebildet sind. Es ergibt sich hiermit ein "Glitzereffekt" auf dem Wasserspiegel und die damit verbundene Reflektion erhöht den Bräunungseffekt der Solarium-Bestrahlung.

Der Liegekomfort und der Erholungseffekt sind durch diese Maßnahmen wesentlich verbessert und erweitert. Nachdem in der Liegematte die Schwimmkörper entweder als separate, voneinander getrennte Teile oder als gelenkig miteinander verbundene Teile ausgebildet sind, passt sich diese Liegematte jedweden Körperbewegungen und Körpererhebungen an. Der damit verbundene Liegeeffekt ist etwa auch mit dem eines Wasserbettes vergleichbar. Es wird hierbei bevorzugt, wenn die Wasserfüllung der Wanne aus erwärmtem Salzwasser besteht, weil hierdurch der Auftrieb für die Liegematte wesentlich erhöht ist. Sie hat hierdurch größere Tragkraft und ist wegen ihrer geringeren Eintauchtiefe daher in horizontaler Ebene leichter beweglich und verschiebbar. Neben dem schwimmenden Gefühl wird hierdurch noch eine in horizontaler Ebene wirkende, schaukelnde Bewegung erzeugt, sofern das Körpergewicht des Benutzers auf der Liegematte verändert wird.

Dem Erholungseffekt ist es weiter zuträglich, wenn im Bereich der Wasserfüllung ein Pulsator angeordnet ist, der elektromotorisch oder mit Membran entsprechende Wasserstösse erzeugt, die gegen die Liegefläche von unten her gerichtet sind. Hierdurch wird noch einVibrations- oder Massageeffekt erzielt.

Zur Erhöhung des optischen Reizes kann das Salzwasser eingefärbt sein oder die Innenwandung der Wanne oder die Liegematte kann beispielsweise durchsichtig mit

0113832

azurblauen Färbung versehen sein, um einen besonderen optischen Effekt zu erzielen.

Die Wanne selbst kann aus Blech, Chromstahl oder einem Kunststoff bestehen, der ggf. auch durchsichtig sein kann.

Der Solarhimmel ist stirnseitig jeweils mit einem Metallbügel versehen, der seinerseits mit seinem unteren Ende stirnseitig an der Wanne befestigt ist. Zwischen dem Solarhimmel und dem oberen Ende des Metallbügels ist ein Schwenklager ausgebildet, welches gestattet, daß der Solarhimmel entweder in eine abgeschwenkte, horizontale Stellung oder eine hochgeschwenkte, vertikale Stellung gebracht werden kann.

Zur Erleichterung der Schwenkbewegung ist zwischen dem beweglichen Teil des Solarhimmels und der Wanne ein Stützarm mit dazwischen angeordneter Gasdruckfeder vorgesehen.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.
Alle in den Unterlagen offenbarten Angaben und Merkmale, insbesondere die in den Zeichnungen dargestellte, räumlicheAusbildung werden als erfindungswesentlich beansprucht, soweit sie einzeln oder in Kombination gegenüber dem Stand der Technik neu sind.

Im folgenden wird die Erfindung anhand von lediglich einen Ausführungsweg darstellende Zeichnungen näher erläutert. Hierbei gehen aus den Zeichnungen und ihrer Beschreibung weitere erfindungswesentliche Merkmale und Vorteile der Erfindung hervor.

0113832

-4-

Es zeigen:

Figur 1 schematisiert gezeichneter Schnitt durch die
Liegefläche mit Solarium-Bestrahlung nach der
Erfindung.

Figur 2 vergrößerte Schnittansicht im Randbereich der
Wanne.

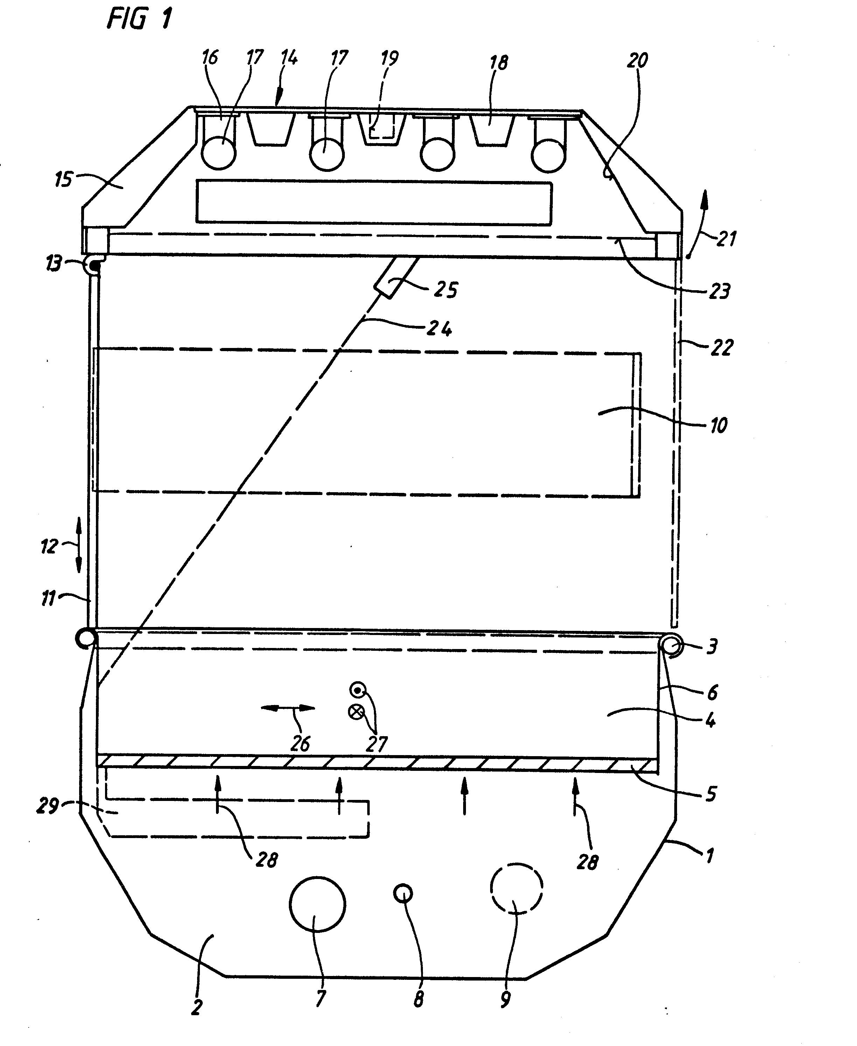

Gemäss Figur 1 besteht die Wanne 1 aus einem geschlossenen Körper aus Blech, Chromstahl oder einem Kunststoff,
der von einer horizontalen Bodenfläche aus in eine
annähernd vertikale Seiten- und Stirn-Wandung übergeht,
und im Übergangsbereich mehrfach abgewinkelt ist.
Bezüglich des Randes 3 sind die Seitenwände der Wanne 1
nach außen hin versetzt, um der darin aufgehängten
Liegematte 5 Bewegung in horizontaler Ebene in den
Pfeilrichtungen 26,27 zu ermöglichen.

. Die Wasserfüllung der Wanne 1 besteht bevorzugt aus
erwärmtem Salzwasser, das mit einer Heizung 7 erwärmt
wird, wobei die Temperatur mit einem Thermostaten 8
eingestellt und überwacht wird. Zusätzlich ist in der
Wanne 1 ein Pulsator 9 eingebaut.

Die Liegematte 5 ist abdichtend und frei auf der Wasseroberfläche der Wanne 1 schwimmend aufgehängt, wobei
sie dicht mit einer umlaufenden, seitlichen Folie 6
verbunden ist, die wiederum dicht an ihrem oberen Rand
mit dem Rand 3 der Wanne 1 verbunden ist. Es ergibt
sich hiermit eine Art von Liegewanne 4, in die sich
der Benutzer hineinlegt, wobei er in beliebiger Weise
auf der Liegematte 5 zu liegen kommt. Die Liegematte
5 wird daher von der Wasserfüllung 2 getragen, wobei
die durch den Pulsator 9 erzeugten Druckwellen in
Pfeilrichtung 28 auf die Unterseite der Liegematte 5 auftreffen.

Oberhalb der Wanne 1 ist schematisiert in der Draufsicht die Grundfläche 10 gezeigt, welche die Liegematte 5 in der Längs- und Quererstreckung bildet.

Jeweils an der vorderen und hinteren Stirnseite der Wanne 1 sind Bügelhalterungen 29 angebracht, an denen jeweils einseitig ein Metallbügel 11 ansetzt, der in nicht näher dargestellter Weise in den Pfeilrichtungen 12 höhenverstellbar ausgebildet ist. Am oberen Ende des Metallbügels 11 ist jeweils ein Schwenklager 13 vorgesehen, das mit seinem anderen, schwenkbaren Ende am Rand des Solarhimmels 14 ansetzt. Hiermit ist der Solarhimmel 14 in der Pfeilrichtung 21 in eine vertikale, hochgeschwenkte Lage klappbar. Zur Unterstützung der Schwenkbewegung ist an der Wanne 1 das eine Ende eines Stützarmes 24 angebracht, der mit einer Gasdruckfeder 25 verbunden ist, die mit dem freien, schwenkbaren Teil des Solarhimmels 14 verbunden ist.

Der Solarhimmel 14 besteht aus einem wannenartigen Gehäuse 15, in dem mehrere Reihen gleichartiger Solarstrahler 17 angeordnet sind. Die Solarstrahler 17 sind in Fassungen 16 gefasst und mit der Unterseite des Solarhimmels 14 verbunden.
Zwischen den Solarstrahlen 17 sind Reflektoren 18 als stegartige Erhöhungen angebracht, wobei im Hohlraum der Reflektoren 18 die jeweiligen Transformatoren 19 für die Solarstrahler 17 angebracht sind. Die Seitenflächen und Stirnflächen des Gehäuses 15 sind mit Reflektionsfolie 20 ausgekleidet, ebenso die Seiten- und Stirnflächen der Reflektoren 18.

An der Unterseite des Solarhimmels 14 ist ein lichtdurchlässiges Schutzgitter 23 angebracht.

An der äußeren und unteren Seite des Gehäuses 15 ist eine rundumlaufende Leiste angebracht, in der ein Vorhang 22 verschiebbar angeordnet ist. Der Vorhang schließt die Liegewanne 4 rundumlaufend ab, so daß der in der Liegewanne 4 auf der Liegematte 5 liegende Benutzer gehäuseartig umschlossen ist.

Der Vorhang 22 kann selbst lichtdurchlässig, gemustert oder lichtundurchlässig sein, und wird an seinen Verbindungskanten mit einem Klettenband verschlossen.

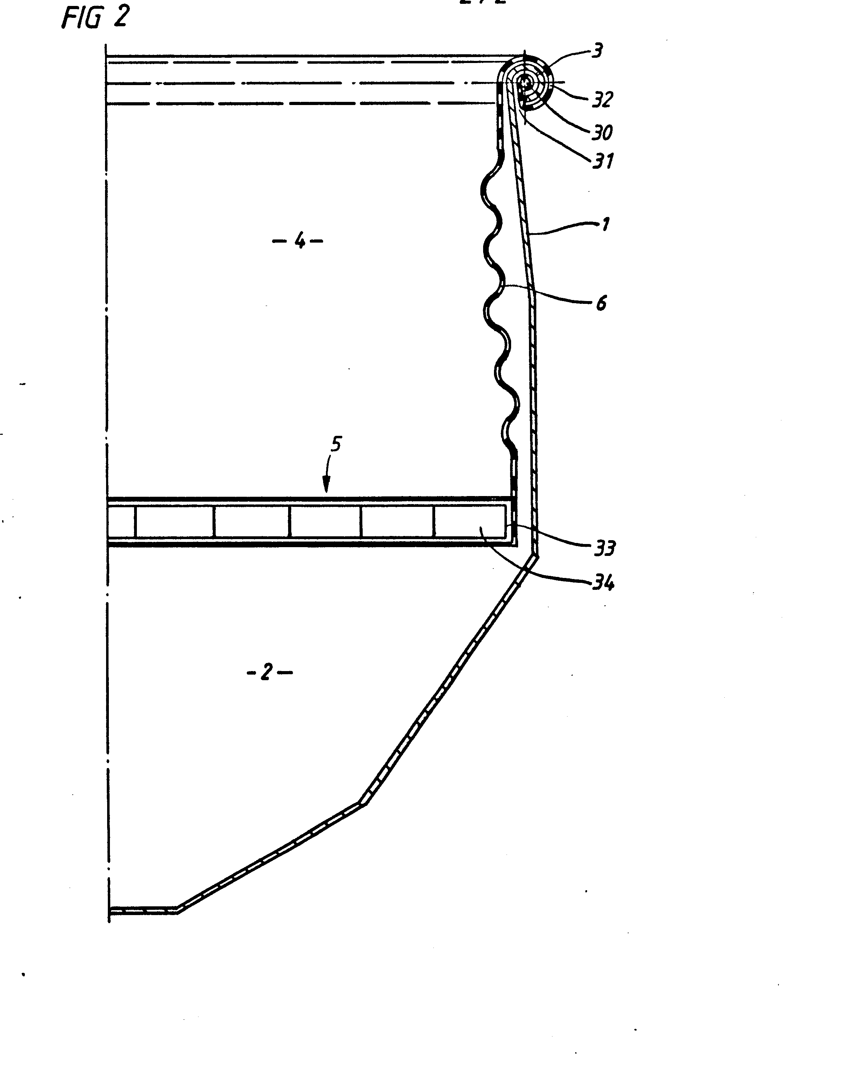

Die Verbindung der Liegematte 5 mit der Wanne 1 erfolgt in der in Figur 2 gezeigten Weise. Die Liegematte 5 besteht zunächst aus einzelnen, durchsichtigen Schwimmkörpern 34, die gelenkig miteinander verbunden sind, oder die durch dicht aneinandergestossen in einen Hohlraum 23 eingebracht sind. Der Hohlraum 23 ist durch die doppelt verschweisste Folie 6 gebildet und ist vollkommen dicht. Die Schwimmkörper 34 können hierbei entweder aus einem durchsichtigen Kunststoff bestehen, wobei die Folie 6 dann ebenfalls durchsichtig wäre; ebenso ist es möglich, daß die Schwimmkörper 34 aus einem geschäumten Kunststoff bestehen, wobei die Folie im Bereich der Liegematte 5 lichtundurchlässig oder lichtdurchlässig sein kann.

An den Seitenwänden ist die Folie 6 hochgezogen und bildet balgartige Wellungen sowohl im belasteten als auch im unbelasteten Zustand. Hierdurch erhält die Liegematte 5 die notwendige Schwingungs- und Bewegungsfreiheit, auch wenn sie mit einer schwergewichtigen Person belastet ist.

Das obere Ende der Folie 6 ist mit dem umgebördelten, umlaufenden Rand 3 der Wanne 1 dadurch verbunden, daß die Folie 6 zunächst eine Umschlingung 32 um den Rand 3 bildet und durch eine unterhalb des Randes angebrachte Längsnut 31 in den Aufnahmeraum des umgebördelten Randes eingreift. In diesem Aufnahmeraum ist ein Kederstab 30 eingeschoben, der einen größeren Durchmesser aufweist, als die größte Weite der Längsnut 31 beträgt, oder Folie wird mit zusätzlichem Rahmen mit dem Bord der Wanne dicht verbunden geklemmt und verschraubt.

Die vorliegende Erfindung ist nicht nur auf die Kombination einer Wanne mit einem Solarhimmel beschränkt; ebenso ist es möglich, herkömmliche Whirlpools mit einer solchen Liegewanne, bestehend aus einer Liegematte 5 mit daran ansetzender Folie 6 zu versehen, wobei die Verbindung zwischen dem umlaufenden Rand des Whirlpools und der Liegewanne 4 mit einer magnetisch wirksamen Klemmleiste oder einer Schraubleiste erfolgen würde. Das gleiche gilt für herkömmliche Badewannen, in die die eben beschriebene Liegewanne 4 eingesetzt werden kann.

Der mit der technischen Lehre nach der vorliegenden Erfindung geschaffene Gegenstand vermittelt einen ganz besonderen Erholungs- und Entmüdungs- und Innovationseffekt, so wie er bei den herkömmlichen Solarstrahlern nicht erreicht werden konnte.

Eine wichtige Möglichkeit besteht noch darin, daß die Wanne 1 als Schaukelbrett z.B. durch entsprechende Lagerung ausgebildet ist.

Mit dieser Maßnahme hat es der Patient in der Hand, nachdem die als Schaukelbrett ausgebildete Wanne bzw. deren Unterlage so gelagert ist, daß der Patient durch Bewegungen in der Vertikalen oder Horizontalen sich selbst eine Wellenbewegung in dem Wasser erzeugen kann, indem er seinen Schwerpunkt entsprechend verlagert, wie man das auf einer Wippe machen könnte. Dadurch kann er eine sich angenehme Wellenbewegung in der Wasserfüllung erzeugen, die einer zusätzlichen Massage oder auch einen beruhigenden Effekt hervorrufen kann. Es ist selbstverständlich, daß man diese Wellenbewegung mit allen anderen ansich bekannten Mitteln, z.B. pulsatorischen Antrieben oder aber durch Exzenterantriebe, erzeugen kann, indem man den Antrieb durch Schwerpunktverlagerung des Patienten durch zusätzliche Antriebe und Hilfsmittel erzeugt. Es ist hier auch als erfindungswesentlich zu betrachten, daß man diese Wellenbewegung, sei sie longitudinal oder transversal, in ihrer Amplitude und Frequenz einstellen kann, je wie es den Bedürfnissen entspricht. Dabei kann man ausgehen von Frequenzen, z.B. mit 50 Herz, durch ein elektromagnetisches Rüttelbrett oder aber,was wohl zweckmässiger sein wird, durch Wellenbewegung, die im Bereich zwischen 1 und 4 Herz liegen.

Patentansprüche
_____

1. Liegefläche mit Solarium-Bestrahlung, wobei ein Solarhimmel höhenverstellbar über einer Liegematte mit etwa rechteckigem Grundriß angeordnet ist, d a d u r c h   g e k e nn z e i c h n e t , daß die Liegematte (5) als schwimmende Fläche abgedichtet und leicht beweglich die Abdeckung einer mit Flüssigkeit gefüllten Wanne (1) bildet.

2. Liegefläche nach Anspruch 1, d a d u r c h   g e k e n n z e i c h n e t , daß die Wanne (1) mit erwärmtem Salzwasser oder Wasser gefüllt ist.

3. Liegefläche nach Anspruch 2, d a d u r c h   g e k e n n z e i c h n e t , daß in die Wasserfüllung der Wanne (1) ein Pulsator (9) ragt.

4. Liegefläche nach Anspruch 1, d a d u r c h   g e k e n n ze i c h n e t , daß die Wanne (1) einen umlaufenden, umgebördelten Rand (3) aufweist, der eine nach unten gerichtete Längsnut (31) aufweist, durch welche hindurch eine Folie (6) greift, die im Bereich des umgebördelten Randes (3) einen Kederstab (30) umschließt oder mit Rahmen mit Bord verklemmt bzw. verschraubt ist.

5. Liegefläche nach Anspruch 1 oder 4, d a d u r c h   g e k e n n z e i c h n e t , daß die Liegematte (5) aus einzelnen Schwimmkörpern (34) besteht, die gelenkig miteinander verbunden in einem Hohlraum (33) angeordnet sind, welcher durch eine (Kunststoff-)Folie (6) gebildet ist, die abdichtend die Schwimmkörper (34) umschließt und ebenfalls dicht mit dem umlaufenden Rand (3) der Wanne (1) verbunden ist.

6. Liegefläche nach Anspruch 1, d a d u r c h
g e k e n n ze   i c h n e t , daß die Liegematte (1)
in allen horizontalen Richtungen (Pfeilrichtungen
26,27) beweglich in der Wanne (1) angeordnet ist.

7. Liegefläche nach Anspruch 1, d a d u r c h
g e k e n n z e i c hn e t , daß der Solarhimmel (14)
einseitig befestigt jeweils über einen Metallbügel (11)
auch höhenverstellbar mit den Stirnseiten der Wanne (1)
verbunden ist.

8. Liegefläche nach Anspruch 7, d a d u r c h
g e k e n n z e i c h n e t , daß der Solarhimmel (14)
in einem Schwenklager (13) am Metallbügel (11) gelagert
ist und aus einer horizontalen Lage in eine vertikale
Lage hochschwenkbar ist.

9. Liegefläche nach Anspruch 8, d a d u r c h
g e k e n n z e i c h n e t , daß zwischen dem schwenkbaren Teil des Solarhimmels (14) und der Wanne (1) ein
Stützarm (24) mit Gasdruckfeder (25) angeordnet ist.

10. Liegefläche nach Anspruch 5, d a d u r c h
g e k e n n z e i c h n e t , daß die Schwimkörper
(34) aus Makrolon und die Folie (6) aus PVC besteht.

11. Liegefläche nach Anspruch 1, d a d u r c h
g e k e n n z e i c h n e t , daß die Wanne (1)
als Schaukelbrett z.B. durch entsprechende Lagerung
ausgebildet ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 911 758 (MUTZHAS)<br><br>* Seite 7, Zeile 13 bis Seite 8, Zeile 10; Ansprüche 2-4,11; Figur 1 * | 1-4,6, 10 | A 61 N 5/06<br>A 47 C 27/00 |
| | --- | | |
| Y | DE-A-3 038 316 (MEYER)<br>* Seite 10, letzter Abschnitt; Seiten 11,12 * | 1,7-9 | |
| | --- | | |
| A | DE-A-2 910 468 (MUTZHAS)<br><br>* Seite 9, Zeilen 6-23; Seite 10, Zeilen 6-9; Figur 4 * | 1,2,10 ,11 | |
| | --- | | |
| A | DE-A-2 537 855 (WOLFF)<br>* Seite 18, Abschnitt 4 * | 1 | RECHERCHIERTE SACHGEBIETE (Int. Cl. 3) |
| | --- | | |
| A | FR-A-2 365 319 (RHONE-POULENC)<br><br>* Seite 1, Zeilen 23-40; Seite 2, Zeilen 8-12 * | 2,3,5, 10 | A 61 N<br>A 47 C |
| | --- | | |
| A | GB-A-1 140 767 (WEINSTEIN)<br>* Seite 3, Zeilen 51-60 * | 2 | |
| | ----- | | |

Der vorliegende Recherchenbericht wurde für alle Patentansprüche erstellt.

| Recherchenort<br>DEN HAAG | Abschlußdatum der Recherche<br>22-03-1984 | Prüfer<br>SIMON J.J.E. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument